# EUROPEAN PATENT SPECIFICATION

(11) **EP 3 080 245 B1**
(45) Date of publication and mention of the grant of the patent: **26.05.2021**
(21) Application number: 14869363.3
(22) Date of filing: 08.12.2014
(51) Int. Cl.: C12N 5/071, C12N 5/07, C12N 5/10

(54) **METHODS FOR THE DETECTION AND QUANTIFICATION OF CIRCULATING ENDOTHELIAL CELLS**
VERFAHREN ZUR DETEKTION UND QUANTIFIZIERUNG VON ZIRKULIERENDEN ENDOTHELZELLEN
PROCÉDÉS DE DÉTECTION ET DE QUANTIFICATION DES CELLULES ENDOTHÉLIALES CIRCULANTES

(30) Priority: 09.12.2013 US 201361913722 P
(43) Date of publication of application: 19.10.2016
(73) Proprietor: The Scripps Research Institute, La Jolla, CA 92037 (US); Kuhn, Peter, Solana Beach, CA 92075 (US); Luttgen, Madelyn, Boston, MA 02114 (US); Kolatkar, Anand, San Diego, CA 92117 (US)
(72) Inventor: KUHN, Peter, Solana Beach, CA 92075 (US); LUTTGEN, Madelyn, Boston, MA 02114 (US); KOLATKAR, Anand, Los Angeles, CA 90007 (US)
(74) Representative: Hutter, Anton
(86) International application number: PCT/US2014/069109
(87) International publication number: WO 2015/088979

(56) References cited:
- US-A1- 2003 108 529
- US-A1- 2012 276 555
- US-A1- 2013 157 347
- NIEVA J ET AL: "High-definition imaging of circulating tumor cells and associated cellular events in non-small cell lung cancer patients: a longitudinal analysis", PHYSICAL BIOLOGY IOP PUBLISHING LTD. UK, vol. 9, no. 1, February 2012 (2012-02), XP002768993, ISSN: 1478-3975
- LI CHUANYIN ET AL: "Detection and Validation of Circulating Endothelial Cells, a Blood-based Diagnostic Marker of Acute Myocardial Infarction", PLOS ONE, vol. 8, no. 3, March 2013 (2013-03), XP002768994, ISSN: 1932-6203
- WOYWODT A ET AL: "Isolation and enumeration of circulating endothelial cells by immunomagnetic isolation: proposal of a definition and a consensus protocol.", March 2006 (2006-03), JOURNAL OF THROMBOSIS AND HAEMOSTASIS : JTH MAR 2006, VOL. 4, NR. 3, PAGE(S) 671 - 677, XP002768995, ISSN: 1538-7933 * the whole document *
- DAMANI, S ET AL.: 'Characterization Of Circulating Endothelial Cells In Acute Myocardial Infarction.' SCIENCE TRANSLATIONAL MEDICINE. vol. 4, no. 126, 21 March 2012, pages 139 - 147, XP008157357 DOI: 10.1126/SCITRANSLMED.3003451
- DATABASE MEDLINE [Online] US NATIONAL LIBRARY OF MEDICINE (NLM), BETHESDA, MD, US; May 2012 (2012-05), KRAAN J ET AL: "A new approach for rapid and reliable enumeration of circulating endothelial cells in patients.", Database accession no. NLM22385979 & KRAAN J ET AL: "A new approach for rapid and reliable enumeration of circulating endothelial cells in patients.", JOURNAL OF THROMBOSIS AND HAEMOSTASIS : JTH MAY 2012, vol. 10, no. 5, May 2012 (2012-05), pages 931-939, ISSN: 1538-7836

## Description

The present disclosure relates generally to methods for the diagnosis of myocardial infarction (MI) and more specifically to methods for the identification and quantification of circulating endothelial cells (CECs).

### BACKGROUND

Coronary artery disease is a leading cause of morbidity and mortality worldwide. The early detection of cardiovascular events is viewed as crucially important for disease prevention and the treatment of patients. However, many cardiovascular events are notoriously difficult to predict. This is especially true for acute myocardial infarction, which involves the rupture of artherosclerotic plaques in a patient's arterial walls. Thus, there exists a significant unmet need for new methods to identify individuals who are at a high imminent risk of suffering cardiovascular events, such as heart attacks or strokes, prior to clinical disease manifestations.

Recently, circulating endothelial cells (CECs) have emerged as promising biomarker candidates for myocardical infarction (MI). Prior to an acute clinical event, inflammation within arterial walls commonly leads to endothelial denudation and the release of CECs into the blood stream. Increased numbers of CECs have been strongly associated with an ongoing MI and thus could represent a potential diagnostic tool in patients presenting with early symptoms.

Currently used methods for CEC identification and quantification include flow cytometry (*e.g.,* FACS) and immunocapture technologies (*e.g.,* CellSearch®). Flow cytometry enables cell sorting but cannot robustly enumerate very small populations of cells, such as CECs, in the presence of much more abundant cell populations, such as the general white blood cell (WBC) population. Certain immunogmagnetic capture platforms have been developed to quantify so-called circulating tumor cells (CTCs) in the blood stream, including the CellSearch® platform, which has been FDA-approved for the monitoring of metastatic cancer patients. Importantly, technologies, like CellSearch®, rely on an initial immunomagnetic bead-based capture step to enrich the rare CTC population prior to its identification and quantification. The CellSearch® CTC immunocapture assay has recently been adapted for CEC detection. The CellSearch® CEC assay requires an initial enrichment step for CD 146-positive white blood cells, which is followed by immunostaining the enriched CTC population for additional cell surface markers, such as CD105 or CD45 (*See, e.g.,* Damani, et al., 2012, Sci. Tansl. Med. 4, 126 ra33). A method for detecting and enumerating circulating endothelial cells in a non-enriched blood sample using the CellSearch® with markers specific for endothelial cells has also been described (Kraan, et al. 2012, Journal of Thrombosis and Haemostasis, 10:931).

However, reported CEC levels in human blood vary greatly across the literature despite substantial assay optimization and standardization efforts. This variability in CEC assay results significantly impedes the further development of CECs as clinically useful biomarkers for MI. The apparent variability in CEC counts is generally thought to be due to highly divergent CEC isolation methods and the variable immunophenotypical definition of CECs. Moreover, CEC capture methods are commonly plagued by a lack of assay sensitivity and specificity.

Thus, there exists a need for improved methods for CEC detection, quantification, and characterization. The present disclosure addresses this need by providing methods for detecting CECs in non-enriched blood samples. Related advantages are provided as well.

### SUMMARY

The present disclosure provides methods for detecting CECs in a non-enriched blood sample.

The invention is as defined in the claims.

In a first aspect of the invention, there is provided a method for detecting circulating endothelial cells (CECs) in a non-enriched blood sample, comprising (a) determining, using fluorescent scanning microscopy, presence or absence of one or more immunofluorescent markers by direct analysis in nucleated cells in the non-enriched blood sample in the presence of surrounding white blood cells (WBCs), wherein the immunofluorescent markers comprise a marker specific for endothelial cells, and (b) assessing the morphology of the nucleated cells, wherein the CECs are detected among the nucleated cells based on a combination of distinct immunofluorescent staining and morphological characteristics.

In one aspect, the disclosure provides a method for detecting circulating endothelial cells (CECs) in a non-enriched blood sample, comprising (a) determining presence or absence of one or more immunofluorescent markers in nucleated cells in the non-enriched blood sample, and (b) assessing the morphology of the nucleated cells, wherein the CECs are detected among the nucleated cells based on a combination of distinct immunofluorescent staining and morphological characteristics.

In some embodiments, the method is performed by fluorescent scanning microscopy. In certain embodiments, the microscopy provides a field of view comprising both CECs and more than 200 surrounding white blood cells (WBCs).

In some embodiments, the immunofluorescent makers comprise a marker specific for white blood cells (WBCs). In some embodiments, one or more of the immunofluorescent markers comprise cluster of differentiation (CD) 45, CD 146 or Von Willebrand factor (vWF).

In some embodiments, the morphological assessment comprises comparing the morphological characteristics of CECs with the morphological characteristics of surrounding WBCs. In some embodiments, the method further comprises assessing the aggregation characteristics of CECs.

Also provided in this disclosure is a method of classifying a subject as a myocardial infarction (MI) patient or a healthy control comprising detecting circulating endothelial cells (CECs) in a subject according to any of the methods provided herein. In certain embodiments, the method at a classification threshold of 0.3 CECs/ml has a specificity of >5%, > 10%, >20%, >30%, > 40%, >50%, >60%, or >70%. In certain embodiments, the method has a specificity >80% at a classification threshold between 1 CEC/ml and 5 CEC/ml. In certain embodiments, the method at a classification threshold of 1.5 CECs/ml has a sensitivity of >80% and a specificity of >95%.

### BRIEF DESCRIPTION OF THE DRAWINGS

**FIG. 1** shows a typical circulating endothelial cell detected with the HD-CEC assay. **FIG. 4 a)** shows a DAPI positive, large nucleus surrounded by numerous smaller nuclei of WBCs. **FIG. 4 b)** shows CD45 positivity on white blood cells only. **FIG. 4 c)** shows a CD146 positive cytoplasm, and **FIG. 4 d)** shows von Willebrand factor positive cytoplasm with a staining pattern that is distinct from the CD146. **FIG. 4 e)** is the composite image of DAPI, CD45 and CD146 and **FIG. 4 f)** is the composite image of DAPI, CD45 and vWF.
**FIG. 2** shows a gallery of representative circulating endothelial cells (CECs) found in MI patients. These cells are characterized by a large DAPI positive nucleus (blue), no CD45 staining (green), and double positive CD146 (red) and vWF (white) staining of cytoplasm.
**FIG. 3** shows result obtained with the HD-CEC assay. Specifically, **FIG. 3** shows CEC counts in healthy controls (C) and in patients that underwent myocardial infaction (MI) or vascular surgery (VS). Counts are reported as HD-CEC/ml blood with mean values highlighted in red.
**FIG. 4** shows CEC aggregates found in MI patients. **FIGs. 4 a)** and **b)** show representative aggregates of 2 and 3 cells, respectively, with DAPI positive nucleus (blue), no CD45 staining (green), and double positive CD146 (red) and vWF (white) staining of cytoplasm. **FIG. 4 c)** shows a frequency histogram showing the distribution of CEC aggregates found in all MI patients.
**FIG. 5** shows ROC curves for the CellSearch® assay (a) and the HC-CEC assay **(b).**
**FIG. 6** shows sensitivity **(a)** and specificity **(b)** performances of HD-CEC and CellSearch® assays at different CEC/ml cut-off points. The difference in specificity correlated inversely with the CEC enumeration threshold.
**FIG. 7** shows a comparison of the overlap between CEC counts in healthy controls (C) and MI patients as determined by the HD-CEC assay and the CellSearch® assay. Y-axis is in log scale and red lines represent median values.

### DETAILED DESCRIPTION

The present disclosure is based, in part, on the unexpected discovery that CECs can be detected in non-enriched blood samples. The present disclosure is further based, in part, on the discovery that CECs can be detected in non-enriched blood samples by combining the detection of one or more immunofluorescent markers in the nucleated cells of a non-enriched blood sample with an assessment of the morphology of the nucleated cells. The present disclosure is futher based, in part, on the discovery that CECs can be detected in non-enriched blood samples by comparing the immunofluorescent marker staining and morphological characteristics of CECs with the immunofluorescent marker staining and morphological characteristics of WBCs.

A fundamental aspect of the present disclosure is the robustness of the disclosed methods. The rare event detection (RED) disclosed herein with regard to CECs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis but that instead compare enriched populations with inherently distorted contextual comparisons of rare events.

The disclosure provides methods for detecting CECs in non-enriched blood samples and for classifying human subjects into MI patients and healthy controls. One major advantage of the present disclosure is the surprisingly high sensitivity with which the methods of this disclosure can classify subjects into MI patients and healthy controls, especially in blood samples having very low CEC counts. High classification sensitivities at low CEC counts facilitate the early detection of otherwise hard to predict cardiovascular events, such as atherosclerotic ruptures and acute myocardial infarction, and thereby facilitate the timely treatment and prevention of such potentially life-threatening events. The present disclosure is therefore of particular benefit to individuals who are at high risk of suffering severe cardiovascular events, *e.g.,* due to a genetic predisposition or certain lifestyle factors, such as a history of smoking, lack of exercise, obesity.

Provided herein are methods for detecting circulating endothelial cells (CEC) in a non-enriched sample, including (a) determining presence or absence of one or more biomarkers in nucleated cells in the non-enriched biological sample, and (b) assessing the morphology of the nucleated cells, whereby the CECs are detected among the nucleated cells based on a combination of distinct biomarker staining and morphological characteristics.

In some embodiments, the disclosure provides a method for detecting circulating endothelial cells (CECs) in a non-enriched blood sample, comprising (a) determining presence or absence of one or more immunofluorescent markers in nucleated cells in the non-enriched blood sample, and (b) assessing the morphology of the nucleated cells, wherein the CECs are detected among the nucleated cells based on a combination of distinct immunofluorescent staining and morphological characteristics.

It must be noted that, as used in this specification and the appended claims, the singular forms "a", "an" and "the" include plural referents unless the content clearly dictates otherwise. Thus, for example, reference to "a biomarker" includes a mixture of two or more biomarkers, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

The biological samples of this disclosure may be any sample suspected to contain CECs, including solid tissue samples, such as bone marrow, and liquid samples, such as whole blood, plasma, amniotic fluid, pleural fluid, peritoneal fluid, central spinal fluid, urine, saliva and bronchial and washes. In some embodiments, the biological sample is a blood sample. As will be appreciated by those skilled in the art, a biological sample can include any fraction or component of blood, without limitation, T-cells, monocytes, neutrophiles, erythrocytes, platelets and microvesicles such as exosomes and exosome-like vesicles.

The biological samples of this disclosure may be obtained from any organism, including mammals such as humans, primates (e.g., monkeys, chimpanzees, orangutans, and gorillas), cats, dogs, rabbits, farm animals (e.g., cows, horses, goats, sheep, pigs), and rodents *(e.g.,* mice, rats, hamsters, and guinea pigs).

It is noted that, as used herein, the terms "organism," "individual," "subject," or "patient" are used as synonyms and interchangeably.

The organism may be a healthy organism or suffer from a disease condition. Disease conditions may include any disease, including cardiovascular diseases such as myocardial infarction (MI; *e.g.,* acute myocardial infarction (MI) or stable coronary artery disease (CAD)), or stroke. In some embodiments, the disease is cancer, diabetes, metabolic syndrome, or an autoimmune disorder. In some embodiments, the healthy or diseased organism is a human organism. In some embodiments, the healthy or diseased organism is an animal model for a disease condition, such as a cardiovascular disease condition. A person of ordinary skill understands that animal models for various disease conditions are well known in the art.

A diseased organism may be untreated or may have received treatment, such as drug treatment (*e.g.,* beta blockers, anti-coagulants (*e.g.,* aspirin, plavix), nitro, heparin, morphine, statins, insulin) or surgery (*e*.*g*., endarterectomy). The drug treatment may predate the sample collection or be ongoing at the time of sample collection.

In some embodiments, the organism is at risk of suffering from myocardial infarction or another cardiovascular disease, as determined by the presence of certain risk factors that are well known in the art. Such risk factors may include, without limitation, genetic predispositions *(e.g.,* for high cholesterol levels, diabetes, obesity, family history of cardiovascular diseases), lifestyle factors (*e*.*g*., cigarette smoking, low exercise, high body/mass index, high fat "western" diet), or clinical disease manifestations (*e*.*g*., atherosclerotic plaques, hypertension, prior medical history of the patient, chest pain, numbness in left arm). In some embodiments, the organism is is a patient who is receiving a clinical workup (*e*.*g*., electrocardiogram (ECG), blood work) to diagnose a heart attack or the risk of a heart attack, such as an imminent heart attack *(e.g.,* a heart attack expected to occur within one week from the time of the clinical workup). In some embodiments, the organism is a patient having elevated blood levels of troponin relative to normal controls.

In some embodiments the sample is a blood sample obtained from a healthy human or a human who is suffering from a cardiovascular disorder, such as MI. In some embodiments the blood sample is from a human MI patient who has received treatment, such as surgery or drug treatment. In some embodiments, the blood sample is obtained from a human who is at risk of suffering MI. In some embodiments, the blood sample is obtained from a human suffering from a buildup of atherosclerotic plaques or any other clinical manifestation of cardiovascular disease well known in the art or who presents with any of the known risk factors for a cardiovascular disese.

The samples of this disclosure may each contain a plurality of cell populations and cell subpopulation that are distinguishable by methods well known in the art *(e.g.,* FACS, immunohistochemistry). For example, a blood sample may contains populations of non-nucleated cells, such as erythrocytes (*e*.*g*., 4-5 million/µl) or platelets (150,000-400,000 cells/µl), and populations of nucleated cells such as WBCs *(e.g.,* 4,500 - 10,000 cells/µl), CECs or CTCs (circulating tumor cells; *e.g.,* 2-800 cells/). WBCs may contain cellular subpopulations of, *e.g.,* neutrophils (2,500-8,000 cells/µl), lymphocytes (1,000-4,000 cells/µl), monocytes (100-700 cells/µl), eosinophils (50-500 cells/µl), basophils (25 - 100 cells/ µl) and the like. The samples of this disclosure are non-enriched samples, *i.e.,* they are not enriched for any specific population or subpopulation of nucleated cells. For example, non-enriched blood samples are not enriched for CECs, WBC, B-cells, T-cells, NK-cells, monocytes, or the like.

The term "rare cell," as used herein, refers to a cell that has an abundance of less than 1:1,000 in a cell population, *e*.*g*., an abundance of less than 1:5,000, 1:10,000, 1:30,000, 1:50:000, 1:100,000, 1:300,000, 1:500,000, or 1:1,000,000. In some embodiments, the rare cell has an abundance of 50:000 to 1:100,000 in the cell population. In some embodiments, the rare cell is a CEC or a CTC.

The samples of this disclosure may be obtained by any means, including, *e*.*g*., by means of solid tissue biopsy or fluid biopsy *(see, e.g.,* Marrinucci D. et al., 2012, Phys. Biol. 9 016003). A blood sample may be extracted from any source known to include blood cells or components thereof, such as venous, arterial, peripheral, tissue, cord, and the like. The samples may be processed using well known and routine clinical methods (*e*.*g*., procedures for drawing and processing whole blood). In some embodiments, a blood sample is drawn into anti-coagulent blood collection tubes (BCT), which may contain EDTA or Streck Cell-Free DNA™. In other embodiments, a blood sample is drawn into CellSave® tubes (Virdex). A blood sample may further be stored for up to 12 hours, 24 hours, 36 hours, 48 hours, or 60 hours before further processing.

In some embodiments, the methods of this disclosure comprise obtaining a white blood cell (WBC) count for the blood sample. In certain embodiments, the WBC count may be obtained by using a HemoCure® WBC device (Hemocure, Ängelholm, Sweden). In some embodiments, the WBC count is used to determine the amount of blood required to plate a consistent loading volume of nucleated cells per slide.

In some embodiments, the methods of this disclosure comprise a step of lysing erythrocytes in the blood sample. In some embodiments, the erythrocytes are lysed, *e.g.,* by adding an ammonium chloride solution to the blood sample. In certain embodiments, a blood sample is subjected to centrifugation following erythrocyte lysis and nucleated cells are resuspended, *e*.*g*., in a PBS solution.

In some embodiments, nucleated cells from a sample, such as a blood sample, are deposited as a monolayer on a planar support. The planar support may be of any material, *e*.*g*., any fluorescently clear material, any material conducive to cell attachment, any material conducive to the easy removal of cell debris, any material having a thickness of < 100 µm. In some embodiments, the material is a film. In some embodiments the material is a glass slide. The glass slide may be coated to allow maximal retention of live cells (*See, e.g.,* Marrinucci D. et al., 2012, Phys. Biol. 9 016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise an initial step of depositing nucleated cells from the blood sample as a monolayer on a glass slide. In certain embodiments, the method comprises depositing about 3 million cells onto a glass slide.

In some embodiments, the glass slide and immobilized cellular samples are available for further processing or experimentation after the methods of this disclosure have been completed.

In some embodiments, the methods of this disclosure comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain embodiments, the fluorescent stain is diamidino-2-phenylindole (DAPI).

The Circulating Endothelial Cells (CECs) of this disclosure are endothelial cells that are circulating in the bloodstream of an organism, such as a human. CECs may further circulate in tissues or fluids outside the bloodstream. According to this disclosure, CECs are detected among the nucleated cells of a sample based on a combination of distinct biomarkers and morphological characteristics.

The biomarkers used to detect CECs may include any biomarker that is specific for an endothelial cell (*e.g.,* cluster of differentiation (CD) 146, Von Willebrand factor (vWF), CD 31, CD 34, or CD 105) or specific for a non-epithelial cell (CD 45).

The term "biomarker" refers to a biological molecule, or a fragment of a biological molecule, the change and/or the detection of which can be correlated with a particular physical condition or state of a CEC. The terms "marker" and "biomarker" are used interchangeably throughout the disclosure. Such biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide

A person skilled in the art will appreciate that a number of methods can be used to determine the presence or absence of a biomarker, including microscopy based approaches, including fluorescence scanning microscopy *(see, e.g.,* Marrinucci D. et al., 2012, Phys. Biol. 9 016003), mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzyme-linked immunosorbant assay (ELISA), immunopercipitation, radioimmunoassay, dot blotting, and FACS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

A person of skill in the art will futher appreciate that he presence or absence of biomarkers may be detected using any class of marker-specific binding reagents known in the art, including, *e*.*g*., antibodies, aptamers, fusion proteins, such as fusion proteins including protein receptor or protein ligand components (*e.g.* CD 146, vWF, CD31, CD 34, CD 105, or CD 45 binding receptors or ligands), or biomarker-specific small molecule binders.

In some embodiments, the presence or absence of vWF, CD145 or CD45 is determined by an antibody.

The antibodies of this disclosure bind specifically to a biomarker. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be monoclonal or polyclonal antibodies. In some embodiments, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used in the methods described herein for direct or indirect detection of the biomarkers in the methods of the disclosure. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the disclosure. Suitable detectable labels include, but are not limited to, fluorescent dyes (*e*.*g*., fluorescein, fluorescein isothiocyanate (FITC), Oregon Green™, rhodamine, Texas red, tetrarhodimine isothiocynate (TRITC), Cy3, Cy5, Alexa Fluor@ 647, Alexa Fluor@ 555, Alexa Fluor® 488), fluorescent markers (*e*.*g*., green fluorescent protein (GFP), phycoerythrin, etc.), enzymes (*e*.*g*., luciferase, horseradish peroxidase, alkaline phosphatase, etc.), nanoparticles, biotin, digoxigenin, metals, and the like.

For mass-sectrometry based analysis, differential tagging with isotopic reagents, *e*.*g*., isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta.-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of ¹²⁵I; or a fluorometer to detect fluorescence in the presence of light of a certain wavelength. If desired, assays used to practice the methods of this disclosure can be automated or performed robotically, and the signal from multiple samples can be detected simultaneously.

In some embodiments, the biomarkers are immunofluorescent markers. In some embodiments, the immunofluorescent makers comprise a marker specific for endothelial cells In some embodiments, the immunofluorescent makers comprise a marker specific for white blood cells (WBCs). In some embodiments, one or more of the immunofluorescent markers comprise cluster of differentiation (CD) 45, CD 146 or Von Willebrand factor (vWF).

In some embodiments, the presence or absence of immunofluorescent markers in nucleated cells, such as CECs or WBCs, results in distinct immunofluorescent staining patterns. Immunofluorescent staining patterns for CECs and WBCs may differ based on which endothelial or WBC markers are detected in the respective cells. Moreover, the immunofluorescent staining pattern for markers identifying the same cell type may be distinct. For example, in CECs the staining patterns for CD 146 and vWF may be both cytoplasmic, but distinct from one another with CD146 staining being diffusely cytoplasmatic and vWF staining containing a punctate pattern. In some embodiments, determining presence or absence of one or more immunofluorescent markers comprises comparing the distinct immunofluorescent staining of CECs with the distinct immunofluorescent staining of WBCs.

In some embodiments, the morphological characteristics comprise nucleus size, nucleus shape, cell size, cell shape, nuclear to cytoplasmic ratio. In some embodiments, the method further comprises analyzing the nucleated cells by nuclear detail, nuclear contour, presence or absence of nucleoli, quality of cytoplasm, quantity of cytoplasm, immunofluorescent staining patterns. In some embodiments, the method further comprises assessing the aggregation characteristics of CECs.

A person of ordinary skill in the art understands that the morphological characteristics of this disclosure may include any feature, property, characteristic, or aspect of a cell that can be determined and correlated with the detection of a CEC.

The methods of this disclosure may be performed with any microscopic method known in the art. In some embodiments, the method is performed by fluorescent scanning microscopy. In certain embodiments the microscopic method provides high-resolution images of CECs and their surrounding WBCs *(see, e.g.,* Marrinucci D. et al., 2012, Phys. Biol. 9 016003)). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some embodiments, microscopic data collection and analysis is conducted in an automated manner.

Typically, each microscopic field contains both CECs and WBCs. In certain embodiments, the microscopic field shows at least 1, 5, 10, 20, 50, or 100 CECs. In certain embodiments, the microscopic field shows at least 10, 25, 50, 100, 250, 500, or 1,000 fold more WBCs than CECs. In certain embodiments, the microscopic field comprises CECs surrounded by at least 10, 50, 100, 150, 200, 250, 500, 1,000 or more WBCs.

In certain embodiments, the microscopy provides a field of view comprising both CECs and more than 200 surrounding white blood cells (WBCs).

In some embodiments, a biomarker is considered "present" in a cell if it is detectable above the background noise of the respective detection method used *(e.g.,* 2-fold, 3-fold, 5-fold, or 10-fold higher than the background; *e*.*g*., 2σ or 3σ over background). In some embodiments, a biomarker is considered "absent" if it is not detectable above the background noise of the detection method used *(e.g.,* < 1.5-fold or <2.0-fold higher than the background signal; *e.g.,* < 1.5σ or <2.0σ over background).

In some embodiments, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CEC-specific immunofluorescent markers, even though absent on WBCs are visible in the WBCs as background signals with fixed heights. Moreover, WBC-specific immunofluorescent markers that are absent on CECs are visible in the CECs as background signals with fixed heights. A cell is considered positive for an immunofluorescent marker *(i.e.,* the marker is considered present) if its fluorescent signal for the respective marker is significantly higher than the fixed background signal (*e*.*g*., 2-fold, 3-fold, 5-fold, or 10-fold higher than the background; *e*.*g*., 2σ or 3σ over background). For example, a nucleated cell is considered CD 45 - positive (CD 45⁺) if its fluorescent signal for CD 45 is significantly higher than the background signal. A cell is considered negative for an immunofluorescent marker *(i.e.,* the marker is considered absent) if the cell's fluorescence signal for the respective marker is not significantly above the background signal (*e*.*g*., < 1.5-fold or <2.0-fold higher than the background signal; *e*.*g*., < 1.5σ or <2.0σ over background).

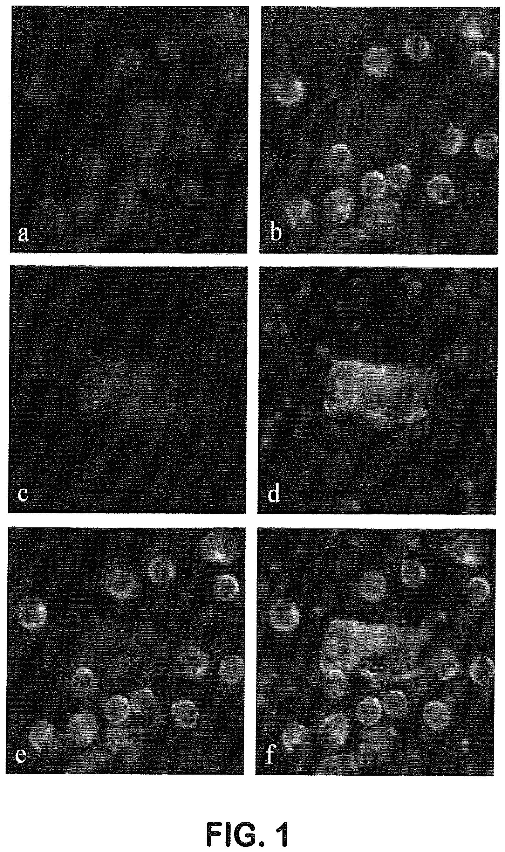

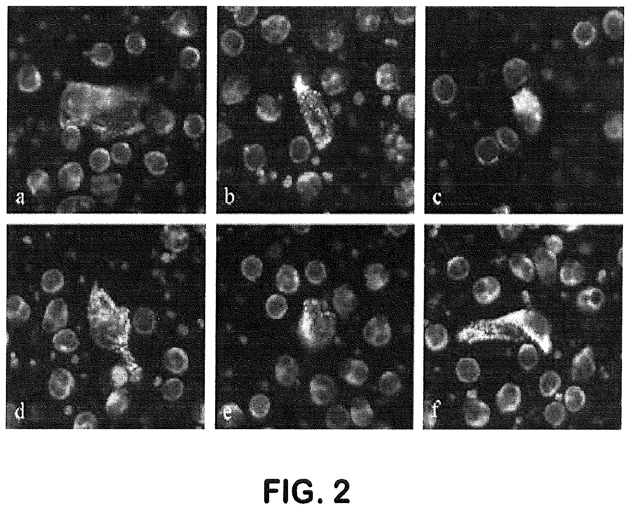

In some embodiments, CECs are identified as nucleated cells *(i.e.,* cells that show above-background fluorescence for a nuclear stain) that are positive for the immunofluorescence markers CD 146 and vWF, but negative for the leukocyte marker CD 45 *(i.e.,* CD 146⁺/vWF⁺/CD 45⁻ cells) *(see, e.g.,* **Example 1,** **FIGs 1** **and** **2****).**

The morphological assessment of a nucleated cell, such as the determination of its size or shape, is based on the fluorescence signals of an immunofluorescent marker. The CECs of this disclosure are morphologically distinct from the surrounding WBCs. In some embodiments, CECs have significantly larger nuclei relative to the nuclei of surrounding WBCs *(see, e.g.,* Experiment 1, **FIGs 1** **and** **2****).** In some embodiments, the morphological assessment comprises comparing the morphological characteristics of CECs with the morphological characteristics of surrounding WBCs.

In some embodiments (also referred to as "high-definition (HD)"-CEC Assay), the comparison of CECs *(i.e.,* target cells of interest) with surrounding WBCs *(i.e.,* negative control cells) improves the performance of the method, *e.g.,* by increasing the accuracy, specificity, or sensitivity of the method, relative to a method wherein no such comparison is performed. In some embodiments, each CEC is surrounded by WBCs. In some embodiments, CECs are compared with surrounding WBCs when determining the presence or absence of a fluorescent marker. In some embodiments, CECs are compared with surrounding WBCs when assessing the morphology of nucleated cells. In some embodiments, CECs are compared with surrounding WBCs when determining the presence or absence of a fluorescent marker and when assessing the morphology of nucleated cells.

The CECs of this disclosure are detected among the nucleated cells of a non-enriched sample based on a combination of distinct immunofluorescent staining and morphological characteristics. In some embodiments, CECs are identified as CD 146⁺/vWF⁺/CD ⁴⁵⁻ cells with significantly enlarged nuclei relative to the WBC cells in the non-enriched sample (*see, e.g.,* **Example 1,** **FIGs 1** **and** **2**).

In some embodiments, the methods of this disclosure include quantifying the number of CECs in a sample (*e*.*g*., a human sample). In some embodiments, the method further comprises quantifying the number of CECs in a blood sample.

In certain embodiments, the methods are used to calculate the concentration of CECs in a sample (*e*.*g*., in [CEC/ml]). For example, CECs are detected in a human blood sample according to the methods of this disclosure. Next, the ratio of CECs to total nucleated cells (*i.e.,* CECs + non-CTCs) is determined for a field of vision. Then, the CEC/total nuclear cell ratio is multiplied by the concentration of total nucleated cells in a blood sample, *e*.*g*., as determined using a standard automated cell counter.

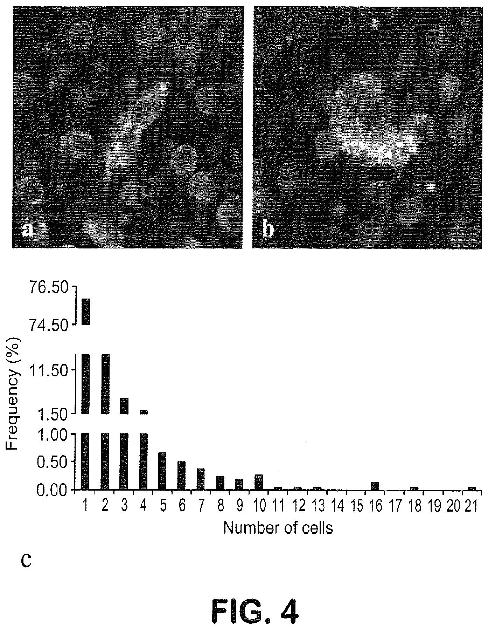

In some embodiments, the methods of this disclosure comprise characterizing the aggregation status of CECs *(see, e.g.,* **Example 3,** **FIG. 4****).** According to the methods of this disclosure, CECs form aggregates of 2, 4, 6, 8, 10, 20, and more cells. CECs may aggregate with other CECs or with surrounding WBCs. According to the methods of this disclosure, the size and frequency of CEC aggregates can be correlated to the health status of subject whose CECs are investigated. In one example, the frequency and size of CEC aggregates is increased in MI patients relative to healthy humans *(see, e.g.,* **Example 3,** **FIG. 4****).**

In other embodiments, the methods of this disclosure comprise the characterization of CEC with respect to any property, characteristic or aspect observable to a person of ordinary skill in the art. Such characteristics may include, morphological characteristics and cellular dynamics *(e.g.,* cell motility, adhesion to extracellular matrix substrates), immunofluorescence characteristics (*e*.*g*., intracellular localization of organelles, biomolecules; formation and localization of biomolecular assemblies, such as lipid rafts), metabolic characteristics (*e*.*g*., energy metabolism, cellular signalling), genomic characteristics (*e*.*g*., genexpression, mRNA splicing), proteomic characteristics (protein expression, localization, post-translational modification). A wide range of methods is available to those skilled in the art to perform a comprehensive characterization of CECs detected using the methods of this disclosure, including, without limitation, mass spectrometry, gene-chips, FISH, immunocytochemistry, fluorescence microscopy, and the like. In some embodiments, the methods of this disclosure allow for the further processing and experimentation on samples after the methods of this disclosure have been completed.

In some embodiments, the methods of this disclosure are used to detect, quantify and characterize CECs in blood samples from human patients suffering from MI.

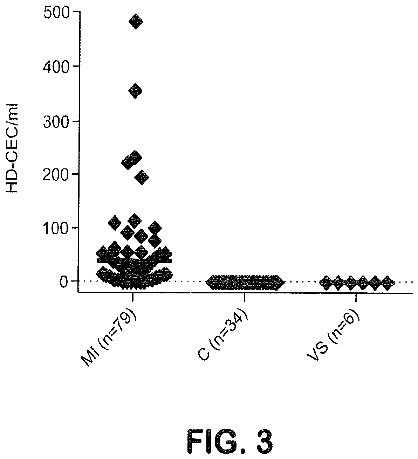

In some embodiments, the methods of this disclosure comprise comparing the relative number of CECs in a healthy subject with the number of CECs in a myocardial infarction (MI) patient. CEC counts in healthy subjects are low. In certain embodiments, the average number of CECs (median or mean) in a healthy subject is < 1 CEC/ml *(see, e.g.,* **Example 2,** **FIG. 3****, Table** 1). By contrast, CEC concentrations in MI patients were determined to be significantly elevated *(e.g.,* one group of MI patients showed a median of about 40 CEC/ml). MI patients who underwent surgery were found to have CEC concentrations similar to healthy controls.

In one example, the methods of this disclosure demonstrated that the morphologies of CECs from MI patients were markedly heterogeneous in appearance. The nuclei from MI patients were larger and aberrantly shaped compared to those detected in control subjects. As discussed above, the CECs from MI patients were determined to aggregate more frequently and in the form of larger-size aggregates *(See, e.g.,* **Example 3,** **FIG. 4****).**

Also provided herein are methods for classifying a subject as a myocardial infaction (MI) patient or a healthy control comprising detecting circulating endothelial cells (CECs) in a subject according to any of the methods disclosed herein.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms; etc.

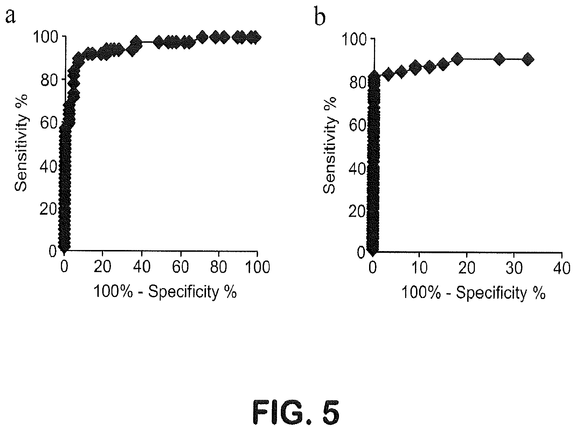

Generally, the methods of this disclosure quantify CECs and classify subjects as patients or healthy controls with great accuracy *(i.e.,* a low rate of false positives or false negatives, such as >90%, >95%, or >99% accuracy; whereby 100% accuracy corresponds to zero false positives and zero false negatives, whereas 50% accuracy corresponds to a random selection), sensitivity *(i.e.,* true MI cases classified as such) and specificity *(i.e.,* true MI controls classified as such). However, it was particularly noteworthy and surprising to find that, in some embodiments, the methods of this disclosure (also referred to as the "HD-CEC assay"), which detect CECs in non-enriched samples and do not require or involve a CEC-specific enrichment step, outperformed the CellSearch® CEC assay, which does rely on a CEC-specific enrichment step. In one example, the HD-CEC assay in direct comparison exhibited much higher specificity than the CellSearch® CEC assay, especially at low CEC concentrations, while maintaining high sensitivity and accuracy *(See, e.g.,* **Example 4,** **FIGs. 5****,** **6** **and** **7****).** This ability of the HD-CEC assay to detect smaller numbers of CECs is especially useful in the prodromal phase of MI, *i.e.,* during the early stages of an impending or ongoing MI event. The detection of smaller numbers of CECs enables the earlier diagnosis of MI and possibly the timely prevention or treatment of a cardiovascular event, thereby preventing severe injury to the patient and possibly death.

Also provided in this disclosure is a method of classifying a subject as a myocardial infarction (MI) patient or a healthy control comprising detecting circulating endothelial cells (CECs) in a subject according to any of the methods provided herein. In some embodiments, the accuracy of the classification is >90%, >95%, or >99%. In some embodiments, the method has a classification threshold (CEC/ml). In certain embodiments, the method at a classification threshold of 0.3 CECs/ml has a specificity of >5%, > 10%, >20%, >30%, > 40%, >50%, >60%, or >70%. In certain embodiments, the method has a specificity >80% at a classification threshold between 1 CEC/ml and 5 CEC/ml. In certain embodiments, the method at a classification threshold of 1.5 CECs/ml has a sensitivity of >80% and a specificity of >95%.

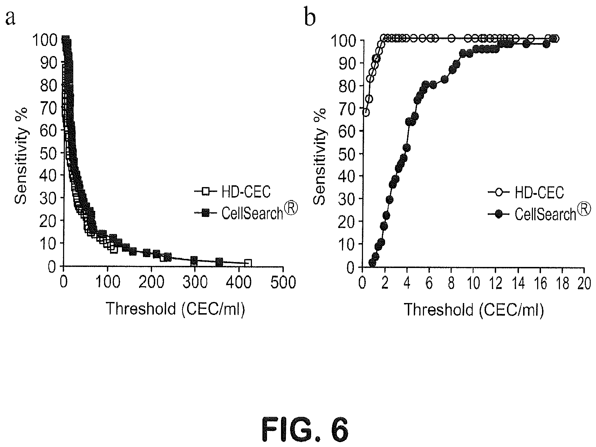

The term "classification threshold," as used herein, refers to a cell count, such as a CEC count *(e.g.,* in cell/ml), for which the characteristics of a method of this disclosure *(e.g.,* the HD-CEC assay), such as accuracy, sensitivity or specificity are being determined (*see, e.g.,* **Example 4,** **FIG. 6****).** For example, the classification threshold may be a cell count (*e.g.,* CEC/ml) at which the sensitivity of the method is high *(e.g.,* >80%) and the specificity is low *(e.g.,* <50%). In another example, the classification threshold may be a cell count at which the sensitivity of the method is low *(e.g.,* < 50%) and the specificity is high. In another example, the classification threshold may be a cell count at which both the sensitivity and the specificity of a method are high *(e.g.,* >80%).

It must be noted that all values provided in the claims include a range including the exact value plus deviations of 1%, 2%, 3%, 4%, or 5%.

In some embodiments, the methods of this disclosure have diagnostic value and are used to assess the efficacy of treatment of MI patients in the form of surgery or drug therapy.

In some embodiments, the methods of this disclosure have prognostic value and are applied to assess the risk of an adverse cardiovascular event, such as rupture of artherosclerotic plaques or acute myocardial infaction. In certain embodiments, the methods are used to determine which patients are at highest or imminent risk of a heart attack.

In some embodiments, the methods of this disclosure are used to screen for drugs or to test the efficacy of drug candidates aimed at the treatment or prevention of acute myocardial infarction.

The following examples are provided by way of illustration, not limitation.

### EXAMPLES

### Example 1. CEC Identification and Characterization Using the HD-CEC Assay

This experiment demonstrates that CEC cells can be characterized and identified using a combination of immunofluorescent markers and morphological characteristics. The HD-CEC assay uses immunofluorescence and morphological measurements to quantify a small number of CECs in the presence of a much larger number of WBCs, for example, in human blood.

### Patient Selection and Blood Sample Collection

Patients enrolled in this study were recruited at 5 hospitals relatively close to the central laboratory at The Scripps Research Institute: Scripps Green, Scripps Mercy, Sharp Memorial, Sharp Grossmont, and Palomar Pomerado hospitals. Institutional review board (IRB) approval was obtained from all recruiting sites, and all patients gave informed consent. Samples were collected from patients with suspected MI at the time of presentation to the emergency room, and were subsequently destroyed if the patient was shown not to have a MI, or if the patient withheld consent. Samples were also excluded if they did not reach the laboratory within the required time or if the sample was otherwise damaged. The final experimental sample set thus consisted of 79 tubes of blood collected from 79 patients subsequently confirmed to have been experiencing a MI at the time of blood collection according to the eligibility criteria which included the usual clinical parameters (*i.e.* CK-MB, troponin, ST elevation) as described by Damani et al. (2012) Sci Transl Med 4 126ra33. In addition, two control groups were collected for the purpose of comparing CEC levels and morphology to such measures obtained from MI patients. Six samples were obtained from patients undergoing endarterectomy procedures (vascular surgery, VS group). Blood was collected after the procedure. This group consisted of patients who by definition had atherosclerotic vascular disease, as evidenced by their need for endarterectomy, and were undergoing an intervention likely to cause disruption of vascular endothelium, but who were not experiencing a MI. Healthy controls were recruited from the normal blood donor program at The Scripps Research Institute. All subjects were well-characterized via self-report. Thirty-four samples were collected from 25 unique healthy donors.

### Blood Sample Preparation for CEC Evaluation

Half of the whole blood volume obtained from 55 subjects enrolled in the study was analyzed in parallel with the HD-CTC assay and the CellSearch® system (Veridex). Peripheral arterial or venous blood analyzed with the HD-CTC assay was drawn into anti-coagulant blood collection tubes (BCT) (EDTA or Streck Cell-Free DNA™). Blood analyzed with the CellSearch® system was drawn into CellSave® tubes (Veridex) and shipped via courier to a central lab. All samples were collected into the corresponding BCT according to the manufacturer's SOP for collection of specimens and stored at room temperature for up to 48 hours before processing.

EDTA or Streck tubes were rocked for 5 minutes before obtaining a white blood cell (WBC) count using a HemoCue® WBC device (Hemocue, Ängelhom, Sweden). The WBC count was used to determine the amount of blood required to plate a consistent loading volume of nucleated cells per slide following the procedures described in Marrinucci et al. (2012) Phys Biol 9 0160. Based upon the WBC count, a volume of blood was subjected to erythrocyte lysis (ammonium chloride solution). After centrifugation, nucleated cells were re-suspended in PBS and attached as a monolayer on custom made glass slides with a proprietary coating that allows maximal retention of live cells. Each slide can hold approximately 3 million nucleated cells, thus the number of cells plated per slide depended on the patients WBC count. This protocol was technically validated and instant assay was shown to be is reproducible and robust across multiple operators and duplicate samples. Cells were fixed with 2% paraformaldehyde, permeabilized with cold methanol, and non-specific binding sites were blocked with 10% goat serum. Slides were subsequently incubated with a cocktail of three primary antibodies consisting of a rabbit derived anti- von Willebrand Factor (vWF; 1:200; Sigma, St. Louis, MO), a mouse-derived anti-CD146 (1:500; Millipore, Billerica, MA), and an Alexa Fluor@ 647 conjugated goat derived anti-CD45 antibody (1:125; AbD Serotec, Oxford, UK). After PBS washes, slides were incubated at 37°C for 20 minutes with a goat anti-mouse Alexa Fluor® 555 and goat anti-rabbit Alexa Fluor@ 488 conjugated secondary antibodies targeting the anti-vWF and anti-CD146 primary antibodies, respectively. Cells were counterstained with DAPI for 10 minutes and mounted with a glycerol-based mounting media. Four slides from each blood sample were evaluated as a single test, which resulted in evaluation of 9.5 ± 1.2 million nucleated cells per test. This represents a range of 0.53 to 3.28 ml of whole blood equivalent per test, depending on the total WBC count of the patient at the time of the draw.

### CEC Identification and Characterization

All four slides from each subject were scanned using a custom made fluorescent scanning microscope equipped with a motorized x/y-stage. Cells were categorized based on signal intensity of all antibodies, morphologic characteristics of the cytoplasm and nucleus, and the staining patterns of the vWF and CD146 antibodies. Candidate cells were nucleated and negative for the WBC marker CD45. Cells in that category were then analyzed for CD146 and vWF intensity followed by a morphologic assessment. Cells were classified as HD-CECs if they were positive for DAPI, CD45 negative, and positive for both CD146 and vWF. Furthermore, they were required to be morphologically distinct from the surrounding WBCs. CECs that had typical apoptotic features like blebbing or fragmentation of the cytoplasm or nucleus were also tracked, although not counted as CECs. Two or more CEC candidates that appeared to have nuclei and/or cytoplasm touching were scored as a CEC aggregate. Each HD-CEC candidate is presented in a field of view with sufficient surrounding WBCs to allow for contextual comparison between cytomorphologic features of the cell in question versus the background WBCs.

### Statistical analysis

Graphical representation and statistical analysis of CEC counts was performed using GraphPad Prism (GraphPad Software, Inc., La Jolla, CA). Differences between the median of two groups were determined using the unpaired t test with Welch's correction for unequal population variances. Differences between more than two groups were determined using Kruskal-Wallis test followed by Dunn's post tests. Repetitive measurements on a single sample were compared using the Wilcoxon matched-pairs signed-rank test. A receiver-operating characteristic (ROC) curve associated with a classifier based on logistic regression was constructed using GraphPad Prism. Graphical representation of CEC threshold vs. specificity and sensitivity was performed using Microsoft Excel (Microsoft, Redmond, WA).

### Results

Representative CECs detected with the HD-CEC assay in blood samples of MI patients are shown in **FIGs 1** and **2****.** The cells were morphologically distinct from the surrounding WBCs. CECs had larger nuclei relative to the other nuclei in the field of view **(****FIG. 1 a)****)** which were those of WBCs, as confirmed by positive CD45 staining **(****FIG. 1 b)****).** Staining patterns for CD146 **(****FIG. 1 c)****)** and vWF **(****FIG. 1 d)****)** were both found to be cytoplasmic, but distinct from one another; CD146 staining was diffusely cytoplasmic, whereas vWF staining was somewhat diffuse with a punctate pattern. Platelets surrounding CECs were also found to be positive for vWF.

Visual inspection of individual fluorescent CEC images revealed that the morphologies of CECs isolated from MI patients were markedly heterogeneous in appearance **(****FIGs 2 a)****-f)).** Different shapes and sizes could be observed. In addition, the nuclei of CECs from MI patients tended to be larger and aberrantly shaped when compared to those detected in control subjects. However, despite their observed heterogeneity in size and shape, CECs were found to be morphologically distinct from surrounding white blood cells.

In conclusion, this experiment demonstrates that CEC cells can be characterized and identified by a combination of immunofluorescent markers and morphological characteristics that clearly distinguish the relatively rare CEC cells from surrounding WBCs. The HD-CEC assay uses these combined immunofluorescence and morphological characteristics to quantify CEC cells in human blood.

### Example 2. CEC Enumeration in Blood Samples Using the HD-CEC Assay

This experiment illustrates that the HD-CEC assay can be used quantitatively to obtain CEC counts, *e*.*g*., in blood from healthy humans and human patients, such as MI patients.

We collected and analyzed 79 peripheral blood draws from 79 MI patients; 28 blood samples were drawn into Streck BCT and 51 into EDTA BCT. We also collected a total of 34 blood samples from 25 healthy controls; 14 samples were drawn into Streck BCT and 20 were drawn into EDTA BCT (*see* **Table 1**).

**Table 1. Results of CEC Enumeration in Blood Samples from MI Patients and Healthy Controls**

| **Study subjects** | **BCT** | **# of blood draws** | **Mean CEC/ml** | **Median CEC/ml** | **Quartiles lower** | **CEC/ml upper** |
|---|---|---|---|---|---|---|
| Myocardial Infarction | EDTA | 51 | 50.3 | 20.5 | 6.3 | 53.1 |
| Myocardial Infarction | Streck | 28 | 23.0 | 4.4 | 1.5 | 24.6 |
| **Myocardial Infarction** | **ALL** | 79 | **40.6** | **13.7** | **2.9** | **39.4** |
| Healthy Control | EDTA | 20 | 0.2 | 0.0 | 0 | 0 |
| Healthy Control | Streck | 14 | 0.4 | 0.2 | 0 | 0.5 |
| **Healthy Control** | **ALL** | **34** | **0.3** | **0.0** | **0** | **0.5** |
| **Vascular Surgery** | **EDTA** | **6** | **0.0** | **0.0** | **0** | **0** |

| | | | | | | |
|---|---|---|---|---|---|---|
| *Breakdown of sample size by study group and blood collection tube (BCT). Mean and median values, as well as lower and upper quartiles are listed for each subject group in the study.* | | | | | | |

CEC counts detected in MI patients whose blood was drawn into EDTA and Streck tubes were not different (*p*>0.05, unpaired t test with Welch's correction). The same pattern was observed in healthy controls. Therefore, data analysis was performed after pooling CEC counts from both tube types used.

CEC counts in MI patients were significantly elevated when compared to healthy controls **(Table 1** and **FIG. 3****,** *p*=3.19x10⁻¹⁴, Kruskal-Wallis test with Dunn's post test). Patients that underwent vascular surgery had lower CEC counts when compared to MI patients but were not significantly different from healthy controls.

CEC enumeration was repeated in 6 randomly selected healthy controls at 2 consecutive visits separated by an 8-week time frame. CEC count per ml blood over the course of the draw schedule did not change (p=0.5, Wilcoxon matched-pairs signed rank test, data not shown), suggesting CEC values remain stable over time in healthy controls.

In summary, this experiment demonstrates that the HD-CEC assay can be used reliably in a quantitative fashion to determine CEC counts in the blood of healthy humans and the blood of MI patients, both pre- and post-surgery.

### Example 3. Detection and Characterization of CEC Aggregates Using the HD-CEC Assay

This experiment illustrates that the HD-CEC assay can detect CECs not only as individual cells, but also in the form of CEC aggregates.

CECs were not only found as individual cells. Many of the CEC images from MI patients often contained several nuclei (blue) associated with one cell body indicated by a continuous double positive CD146 (red) and vWF (white) staining of cytoplasm **(****FIGs. 4 a)** and b)). These images represent aggregates of injured cells released into circulation during plaque rupture.

Aggregates of 2 or more CECs were detected in 65 out of 79 MI patients; 53 of which (81.5%) were found to have CEC aggregates, *i.e.* two or more cells that appeared to have nuclei and/or cytoplasm touching. Measurements of the percentage of CECs in aggregates (that is, total number of CECs in aggregates/total number of CECs) found that 46.3% ± 23.1% of MI patient's CECs reside in aggregates. CEC aggregates in MI patients varied greatly in number of CEC per aggregate. Up to 21 CECs were detected in an aggregate but aggregates containing 2-4 cells were more frequently found **(****FIG. 4 c)****).**

In control subjects, 5 of 34 blood samples (14.7%) had more than one CEC. Two or 3 individual CECs were detected but none of these samples had detectable CEC aggregates.

In summary, this experiment demonstrates that the HD-CEC assay can detect CEC aggregates besides individual CECs. The assay can detect aggregates of as little as two CECs and can reliably detect the largest CEC aggregates observed that contain up to 21 CECs.

### Example 4. Comparison of HD-CEC and CellSearch® Assays Regarding Their Accuracy, Sensitivity, and Specificity in Classifying Human Subjects into MI patients and Healthy Controls

This experiment illustrates that the HD-CEC assay detects CECs with great accuracy, sensitivity, and specificity. Surprisingly, the HD-CEC assay outperformed the CellSearch® assay platform by a wide margin in terms of the specificity of CEC detection.

The CellSearch® system isolates enriched populations of CECs from blood using anti-CD146 antibody conjugated magnetic nanoparticles. CD146⁺ enriched cells are then fluorescently labeled with the nuclear stain DAPI and antibodies against CD 105 and CD45. CECs are identified based on three criteria: positive staining for nuclei, positive staining for CD105 (an endothelial cell marker), and negative staining for CD45 (*see* Damani et al. (2012) Sci Transl Med 4 126ra33).

Some of samples analyzed with the HD-CEC assay were collected as part of a larger study that involved split samples from the same phlebotomy. Half of the whole blood volume obtained from these 55 subjects was analyzed separately with the CellSearch® system and described by Damani *et al..*

A quantitative comparison of sensitivity and specificity measures of the HD-CEC assay vs. the CellSearch® assay was conducted by constructing receiver-operating characteristic (ROC) curves associated with a logistic regression classifier with a 10 fold cross-validation performed for each assay, and by measuring the total area under the ROC curves (FIG. 5). CellSearch® analysis included CEC/ml counts in 94 samples (44 healthy controls and 55 STEMI patients) as reported by Damani *et al.* The HD-CEC assay included CEC/ml counts in 34 healthy controls and 79 MI patients (55 patients evaluated by Damani *et al.* with the CellSearch® assay and 24 additional patients) accounting for a total of 113 samples.

Calculated areas under the curve (AUC) for the HD-CEC assay and the CellSearch® assay data were 93.3% and 95.4%, respectively. An AUC equal to 100% suggests perfect classification (zero false positives and zero false negatives), whereas an AUC of 50% indicates random selection. The AUC determined for the two assays were not statistically different (p>0.05), indicating that both assays have similar overall accuracy in discriminating MI patients from healthy controls. However, considering the cut-off point for discrimination between MI patients and control cases, it was found that the HD-CEC assay, as implemented here, had a significantly higher specificity (that is, true controls classified as such) while maintaining the necessary sensitivity (that is, true MI cases classified as such). A classification threshold of 1.5 CECs/ml was associated with 83.5% sensitivity and 97% specificity for correctly classifying an MI case or control.

**FIG. 6** shows sensitivity and specificity of each assay at different cut-off values of CEC/ml count. Sensitivity performances for both assays overlapped **(****FIG. 6 a)****),** whereas specificity performances clearly differed at low CEC/ml cut-off points **(****FIG. 6 b)****).**

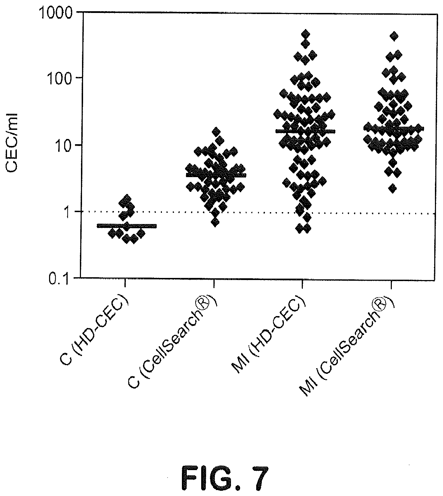

The difference in specificity between the two assays was found to correlate inversely with CEC enumeration threshold. For example at 0.3 CEC/ml count (mean CEC/ml count in control subjects, **Table 1),** the HD-CEC assay demonstrated 70% specificity, while the specificity of the CellSearch® assay for the same count was below 2%. Moreover, the HD-CEC assay demonstrated >80% specificity at CEC counts >1 CEC/ml and >90% specificity at CEC counts >2 CEC/ml (cp. CellSearch® specificity <5% at 1 CEC/ml; <15% at 2 CEC/ml) **(****FIG. 6b**)). Overall, the HD-CEC assay showed superior sensitivities to the CellSearch® assay for CEC counts below 8 CEC/ml **(****FIG. 6b****)).** The superior specificity of the HD-CEC assay is due to much lower overlap in CEC/ml enumeration in MI patients and control subjects as compared with the CellSearch® assay **(****FIG. 7****).** The average CEC/ml count in healthy controls detected by the HD-CEC assay (0.26 ± 0.08, SE) was significantly lower than the count according to the CellSearch® assay (4.38 ± 0.48, SE) (*p*<0.0001, Mann Whitney test).

In summary, this experiment demonstrates that the HD-CEC assay detects CECs in blood samples with great accuracy, sensitivity, and specificity. Surprisingly, the HD-CEC assay outperforms the standard CellSearch® assay by a wide margin with respect to sensitivity, especially at low CEC counts.

## Claims

1. A method for detecting circulating endothelial cells (CECs) in a non-enriched blood sample, comprising (a) determining, using fluorescent scanning microscopy, presence or absence of one or more immunofluorescent markers by direct analysis in nucleated cells in the non-enriched blood sample in the presence of surrounding white blood cells (WBCs), wherein the immunofluorescent markers comprise a marker specific for endothelial cells, and (b) assessing the morphology of the nucleated cells, wherein the CECs are detected among the nucleated cells based on a combination of distinct immunofluorescent staining and morphological characteristics.

2. The method of claim 1, wherein said immunofluorescent markers comprise a marker specific for white blood cells (WBCs).

3. The method of claim 1 comprising an initial step of identifying said nucleated cells in the non-enriched blood sample, preferably wherein said nucleated cells are identified with a fluorescent stain.

4. The method of claim 1, wherein said immunofluorescent markers specific for endothelial cells comprise cluster of differentiation CD 146, Von Willebrand factor (vWF), CD 31, CD 34, or CD 105.

5. The method of claim 4, wherein said immunofluorescent markers comprise CD 146 or vWF.

6. The method of claim 2, wherein said immunofluorescent marker comprises cluster of differentiation (CD) 45.

7. The method of claim 1, wherein said morphological assessment comprises comparing the morphological characteristics of CECs with the morphological characteristics of surrounding WBCs.

8. The method of claim 1, wherein said determining presence or absence of one or more immunofluorescent markers comprises comparing the distinct immunofluorescent staining of CECs with the distinct immunofluorescent staining of WBCs.

9. The method of claim 1, wherein said morphological characteristics comprise nucleus size, nucleus shape, cell size, cell shape, or nuclear to cytoplasmic ratio.

10. The method of claim 1, further comprising analyzing said nucleated cells by nuclear detail, nuclear contour, presence or absence of nucleoli, quality of cytoplasm, quantity of cytoplasm, or immunofluorescent staining patterns.

11. The method of claim 1, further comprising assessing the aggregation characteristics of CECs.

12. The method of claim 1, further comprising the initial step of depositing nucleated cells from the blood sample as a monolayer on a glass slide.

13. The method of claim 8, wherein the fluorescent stain comprises a nucleic acid specific stain.

14. The method of claim 1, further comprising quantifying the number of CECs in the blood sample, preferably wherein the average number of CECs (median or mean) is < 1 CEC/ml.

15. The method of claim 1, further comprising comparing the relative number of CECs in a healthy subject with the number of CECs in a myocardial infarction (MI) patient.

16. A method of classifying a subject as a myocardial infarction (MI) patient or a healthy control comprising detecting circulating endothelial cells (CECs) in a subject according to the method of any one of claims 1 to 15.

## Patentansprüche

1. Verfahren zum Detektieren von zirkulierenden Endothelzellen (CECs) in einer nicht angereicherten Blutprobe, umfassend (a) Bestimmen, unter Verwendung von Fluoreszenz-Rastermikroskopie, einer Anwesenheit oder Abwesenheit eines oder mehrerer Immunfluoreszenzmarker durch direkte Analyse in nukleierten Zellen in der nicht angereicherten Blutprobe in der Anwesenheit von umgebenden weißen Blutzellen (WBCs), wobei die Immunfluoreszenzmarker einen spezifischen Marker für Endothelzellen umfassen, und (b) Bewerten der Morphologie der nukleierten Zellen, wobei die CECs unter den nukleierten Zellen basierend auf einer Kombination unterschiedlicher Immunfluoreszenzfärbung und morphologischen Eigenschaften detektiert werden.

2. Verfahren nach Anspruch 1, wobei die Immunfluoreszenzmarker einen spezifischen Marker für weiße Blutzellen (WBCs) umfassen.

3. Verfahren nach Anspruch 1, umfassend einen anfänglichen Schritt eines Identifizierens der nukleierten Zellen in der nicht angereicherten Blutprobe, wobei die nukleierten Zellen vorzugsweise mit einer Fluoreszenzfärbung identifiziert werden.

4. Verfahren nach Anspruch 1, wobei die für Endothelzellen spezifischen Immunfluoreszenzmarker Unterscheidungsgruppen (Cluster of Differentiation, CD) CD 146, Von-Willebrand-Faktor (vWF), CD 31, CD 34 oder CD 105 umfassen.

5. Verfahren nach Anspruch 4, wobei die Immunfluoreszenzmarker CD 146 oder vWF umfassen.

6. Verfahren nach Anspruch 2, wobei der Immunfluoreszenzmarker Unterscheidungsgruppe (Cluster of Differentiation, CD) 45 umfasst.

7. Verfahren nach Anspruch 1, wobei die morphologische Bewertung ein Vergleichen der morphologischen Eigenschaften der CECs mit den morphologischen Eigenschaften von umgebenden WBCs umfasst.

8. Verfahren nach Anspruch 1, wobei das Bestimmen einer Anwesenheit oder Abwesenheit von einem oder mehreren Immunfluoreszenzmarkern ein Vergleichen der unterschiedlichen Immunfluoreszenzfärbung von CECs mit der unterschiedlichen Immunfluoreszenzfärbung von WBCs umfasst.

9. Verfahren nach Anspruch 1, wobei die morphologischen Merkmale Kerngröße, Kernform, Zellgröße, Zellform oder ein Verhältnis von Kern zu Zytoplasma umfassen.

10. Verfahren nach Anspruch 1, ferner umfassend ein Analysieren der kernhaltigen Zellen nach Kerndetail, Kernkontur, Anwesenheit oder Abwesenheit von Nukleoli, Qualität von Zytoplasma, Quantität von Zytoplasma oder Immunfluoreszenzfärbungsmustern.

11. Verfahren nach Anspruch 1, ferner umfassend ein Bewerten der Aggregationseigenschaften von CECs.

12. Verfahren nach Anspruch 1, ferner umfassend den anfänglichen Schritt eines Aufbringens nukleierter Zellen aus der Blutprobe als Monoschicht auf einem Glasträger.

13. Verfahren nach Anspruch 8, wobei die Fluoreszenzfärbung eine nukleinsäurespezifische Färbung umfasst.

14. Verfahren nach Anspruch 1, ferner umfassend ein Quantifizieren der Anzahl von CECs in der Blutprobe, vorzugsweise wobei die durchschnittliche Anzahl von CECs (Median oder Mittelwert) < 1 CEC/ml ist.

15. Verfahren nach Anspruch 1, ferner umfassend ein Vergleichen der relativen Anzahl von CECs bei einem gesunden Subjekt mit der Anzahl von CECs bei einem Myokardinfarkt-Patienten (MI-Patienten).

16. Verfahren zum Klassifizieren eines Subjekts als Myokardinfarkt-Patient (Mi-Patient) oder als gesunde Kontrolle, umfassend ein Detektieren von zirkulierenden Endothelzellen (CECs) bei einem Subjekt gemäß dem Verfahren nach einem der Ansprüche 1 bis 15.

## Revendications

1. Procédé de détection de cellules endothéliales circulantes (CEC) dans un échantillon de sang non enrichi, comprenant (a) la détermination, à l'aide d'une microscopie à balayage fluorescent, la présence ou l'absence d'un ou plusieurs marqueurs immunofluorescents par analyse directe dans des cellules nucléées dans l'échantillon de sang non enrichi en présence des globules blancs (WBC) environnants, lesdits marqueurs immunofluorescents comprenant un marqueur spécifique des cellules endothéliales, et (b) l'évaluation de la morphologie des cellules nucléées, lesdites CEC étant détectées parmi les cellules nucléées sur la base d'une combinaison de coloration immunofluorescente distincte et de caractéristiques morphologiques.

2. Procédé selon la revendication 1, lesdits marqueurs immunofluorescents comprenant un marqueur spécifique des globules blancs (WBC).

3. Procédé selon la revendication 1, comprenant une étape initiale d'identification desdites cellules nucléées dans l'échantillon de sang non enrichi, de préférence lesdites cellules nucléées étant identifiées avec un colorant fluorescent.

4. Procédé selon la revendication 1, lesdits marqueurs immunofluorescents spécifiques des cellules endothéliales comprenant un cluster de différenciation CD 146, facteur Von Willebrand (vWF), CD 31, CD 34 ou CD 105.

5. Procédé selon la revendication 4, lesdits marqueurs immunofluorescents comprenant le CD 146 ou le vWF.

6. Procédé selon la revendication 2, ledit marqueur immunofluorescent comprenant le cluster de différenciation (CD) 45.

7. Procédé selon la revendication 1, ladite évaluation morphologique comprenant la comparaison des caractéristiques morphologiques des CEC aux caractéristiques morphologiques des WBC environnants.

8. Procédé selon la revendication 1, ladite détermination de la présence ou de l'absence d'un ou plusieurs marqueurs immunofluorescents comprenant la comparaison de la coloration immunofluorescente distincte des CEC à la coloration immunofluorescente distincte des WBC.

9. Procédé selon la revendication 1, lesdites caractéristiques morphologiques comprenant la taille du noyau, la forme du noyau, la taille de la cellule, la forme de la cellule ou le rapport nucléaire/cytoplasmique.

10. Procédé selon la revendication 1, comprenant en outre l'analyse desdites cellules nucléées par les détails nucléaires, le contour nucléaire, la présence ou l'absence de nucléoles, la qualité du cytoplasme, la quantité de cytoplasme ou les motifs de coloration immunofluorescente.

11. Procédé selon la revendication 1, comprenant en outre l'évaluation des caractéristiques d'agrégation des CEC.

12. Procédé selon la revendication 1, comprenant en outre l'étape initiale de dépôt de cellules nucléées provenant de l'échantillon de sang sous la forme d'une monocouche sur une lamelle de verre.

13. Procédé selon la revendication 8, ledit colorant fluorescent comprenant un colorant spécifique d'acide nucléique.

14. Procédé selon la revendication 1, comprenant en outre la quantification du nombre de CEC dans l'échantillon de sang, de préférence le nombre moyen de CEC (médian ou moyen) étant < 1 CEC/ml.

15. Procédé selon la revendication 1, comprenant en outre la comparaison du nombre relatif de CEC chez un sujet en bonne santé au nombre de CEC chez un patient souffrant d'un infarctus du myocarde (IM).

16. Procédé de classification d'un sujet en tant que patient souffrant d'un infarctus du myocarde (IM) ou témoin en bonne santé comprenant la détection de cellules endothéliales circulantes (CEC) chez un sujet conformément au procédé selon l'une quelconque des revendications 1 à 15.
